(19) Europäisches Patentamt
European Patent Office
Office européen des brevets

(11) Numéro de publication: **0 220 112 B1**

(12) **FASCICULE DE BREVET EUROPEEN**

(45) Date de publication de fascicule du brevet: **08.04.92**

(21) Numéro de dépôt: **86402236.3**

(22) Date de dépôt: **09.10.86**

(51) Int. Cl.5: **C07C 215/00**, C07D 295/08, C07D 211/14, C07D 217/04, A61K 31/135, A61K 31/395, A01N 33/08, A61K 7/00, A23L 3/34

# (54) Dérivés aromatiques substitués par un groupement (oméga-amino) alcanol à activité antimicrobienne, leur préparation et les compositions les contenant.

(30) Priorité: **11.10.85 FR 8515102**

(43) Date de publication de la demande: **29.04.87 Bulletin 87/18**

(45) Mention de la délivrance du brevet: **08.04.92 Bulletin 92/15**

(84) Etats contractants désignés: **AT BE CH DE ES FR GB IT LI LU NL SE**

(56) Documents cités: **BE-A- 642 084** **US-A- 3 852 363**

(73) Titulaire: **SANOFI**
**40, Avenue George V**
**F-75008 Paris(FR)**

(72) Inventeur: **Proietto, Vincenzo**
**1, cour du Merle**
**F-34680 Saint Georges d'Orques(FR)**
Inventeur: **Mosse, Madeleine**
**La Chanterelle Chemin du Réservoir de Montmaur**
**F-34100 Montpellier(FR)**
Inventeur: **DeMarne, Henri**
**Le Florence 65, avenue Major Flandre**
**F-34000 Montpellier(FR)**

(74) Mandataire: **Gillard, Marie-Louise et al**
**Cabinet Beau de Loménie 55, Rue d'Amsterdam**
**F-75008 Paris(FR)**

EP 0 220 112 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**Description**

La présente invention a pour objet de nouveaux dérivés aromatiques substitués par un groupement (oméga-amino) alcanol. Ces composés ont une activité antimicrobienne.

La présente invention concerne également l'utilisation des composés selon l'invention dans des compositions à usage antiseptique, antimicrobien, comme désinfectants ou conservateurs, notamment dans les domaines de la pharmacie, la cosmétologie ou l'agroalimentaire.

Par un autre de ses aspects, la présente invention se réfère au procédé de préparation des composés selon l'invention.

L'activité pharmacologique de certains dérivés du naphtalène est connue dans la littérature. Ainsi dans J. Med. Chem., 1965, 8, 589, S. CASADIO, G. PALA et col. ont montré que le naphtyl-1 acétonitrile présente une activité analgésique, anti-inflammatoire et antispasmodique. Plus tard, G. PALA et col., dans J. Med. Chem., 1966, 9, 603, ont étudié les dérivés alpha substitués de l'acide naphtyl-1 acétique et ils ont mis en évidence leurs propriétés cholérétiques et hypoglycémiantes et l'absence d'activité antibactérienne ou antifongique in vitro. R. K. ZAHN et col., ont décrit dans Nature, 1966 (212), 5059, 298, que le naphtyl-2 éthanol inhibe la division cellulaire des cellules de lymphome de souris. Enfin, le (méthoxy-6 naphtyl-2)-2 méthyl-2 éthanol sous sa forme (-) est un anti-inflammatoire connu dénommé naproxol (brevet français 2 068 539).

Par ailleurs, des dérivés phénylalcanols de formule :

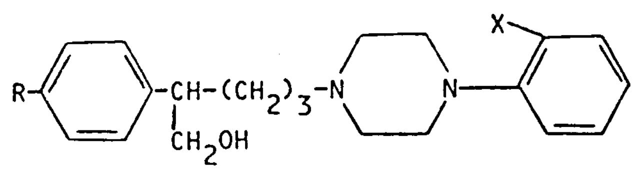

sont décrits dans le brevet belge n° 642 084 pour leur utilisation dans le traitement des désordres neurologiques.

De façon tout à fait surprenante, on a maintenant trouvé que les composés de l'invention, caractérisés par la formule (I) développée ci-après, présentent une activité antimicrobienne mise en évidence par leur pouvoir bactéricide et fongicide.

Des composés similaires à ladite formule (I) mais dans laquelle Alk représente un groupe méthylène ont été décrits dans :

- le brevet GB-A-1 434 826
- Chem. Ber. 1968, 101, 1120-1130 (Metzger et al.)
- J. Org. Chem. 1959, 29, 1928-1936 (testa et al.)
- Coll. Czech. Chem. Commun. 1981, 46, 597-605 (Sindeler et al.).

Dans aucun de ces documents, on ne décrit ni ne suggère, pour lesdits composés, une activité antimicrobienne.

La présente invention a pour objet de nouveaux dérivés aromatiques de formule :

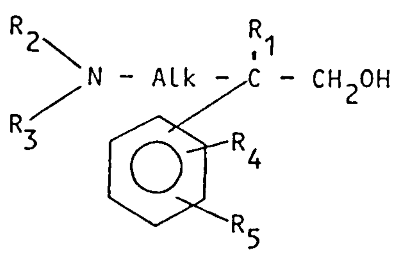

(I)

dans laquelle :

| | |
|---|---|
| Alk | représente un groupe alkylène de 2 à 10 atomes de carbone ; |
| $R_1$ | représente l'hydrogène ou un alkyle comportant de 1 à 6 atomes de carbone ; |
| $R_2$ et $R_3$ | sont semblables ou différents et représentent un cycloalkyle de 3 à 6 atomes de carbone ou un alkyle droit ou ramifié comportant de 1 à 6 atomes de carbone non substitué ou substitué par un groupement phényle ou méthylphényle ; ou $R_2$ et $R_3$ forment ensemble |

avec l'atome d'azote auquel ils sont liés un hétérocycle monoazoté ne contenant pas d'autre hétéroatome, tel que pyrrolidino, pipéridino, azépino, hexaméthylèneimino, méthyl-4 pipéridino, benzyl-4 pipéridino, phényl-4 pipéridino, tétrahydro-1,2,3,4 isoquinolyl-2 ;

- $R_4$ représente un hydrogène, un halogène, un méthyle ou un phényle ;
- $R_5$ représente un hydrogène, un halogène ou un méthyle ;
- ou $R_4$ et $R_5$ pris ensemble avec le noyau benzénique auquel ils sont liés constituent un groupement naphtyl-1 ou naphtyl-2 ;

ainsi que les sels pharmaceutiquement acceptables avec des acides minéraux ou organiques.

Le procédé de préparation des composés selon l'invention est caractérisé en ce que l'on réduit l'acide de formule :

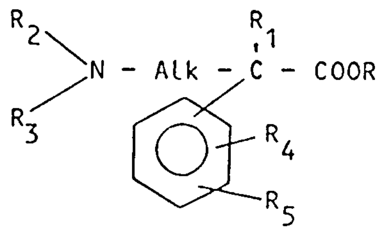

(II)

dans laquelle Alk, $R_1$, $R_2$, $R_3$, $R_4$, $R_5$ ont les significations indiquées précédemment et R représente l'hydrogène ou un groupe alkyle. La réduction est réalisée par des moyens connus tels que l'action d'un agent réducteur ou une réaction d'électrolyse en milieu acide. Comme agent réducteur, on utilise un hydrure métallique, éventuellement en présence d'un catalyseur. Plus particulièrement, la réduction de l'acide ou de l'ester (II) pour obtenir le composé selon l'invention peut être réalisée par l'hydrure de bore, par l'hydrure d'aluminium, par le borohydrure de lithium, par le borohydrure d'aluminium, par le borohydrure de sodium, par l'hydrure de sodium et d'aluminium, par l'hydrure de lithium et d'aluminium ou par un autre hydrure du bore tel que le diméthylsulfure de borane, ou le benzodioxaborole-1,3,2. De façon préférentielle, la réduction est réalisée sur l'acide (R = H) ou sur l'ester éthylique ($R = C_2H_5$) par action du Vitride® (hydrure de sodium bis (méthoxy-2 éthoxy) aluminium) dans un solvant inerte tel que le benzène ou le toluène à une température ambiante ou à une température comprise entre la température ambiante et 80 ° C. Le composé obtenu est isolé selon les méthodes habituelles par exemple par simple précipitation et éventuellement transformé en un sel avec pharmaceutiquement acceptables des acides minéraux ou organiques.

Les acides et leurs esters (II) sont préparés par des méthodes connues. On utilise comme produit de départ du phényl-acétonitrile substitué sur le noyau benzénique par $R_4$ et $R_5$ ou le naphtyl-1 acétonitrile ou le naphtyl-2 acétonitrile selon le composé attendu, sur lequel on fait agir de l'amidure de sodium dans un solvant inerte porté à reflux et on ajoute une choroalkylamine de formule :

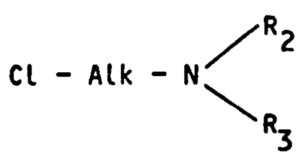

(III) ;

on chauffe au reflux du solvant pour former le composé :

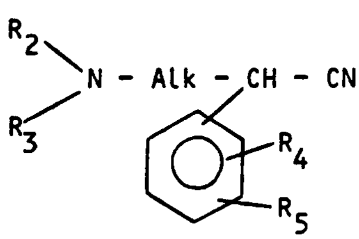

(IV) ;

lorsque le substituant $R_1$ est un alkyle, le nitrile (IV) est alkylé par action de l'amidure de sodium et d'un halogénure $R_1X$ pour obtenir le nitrile :

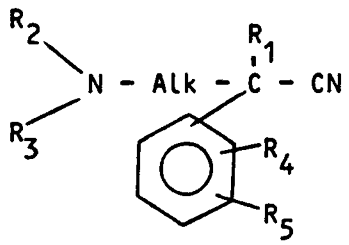

(V) ;

on hydrolyse le nitrile (IV ou V) en milieu acide fort (pH inférieur à 2) pour obtenir l'acide correspondant, qui est éventuellement estérifié par un groupe alkyle R selon des méthodes connues.

L'activité bactéricide des produits selon l'invention a été étudiée sur différentes souches par la méthode décrite ci-après :

Un inoculum bactérien est mis en contact avec différentes dilutions du produit à tester et, ce, pendant un temps limité. A la fin du contact, une partie aliquote du mélange suspension bactérienne/produit est déposée à la surface d'un milieu de culture gélosé contenant un neutralisant de l'activité antibactérienne du produit. La concentration bactéricide retenue est la concentration minimale de produit à partir de laquelle les bactéries ne poussent plus. Cette concentration est exprimée en µg/ml.

Les souches bactériennes choisies pour l'étude sont :

1 - Escherichia Coli CNCM 54125 ;
2 - Klebsiella pneumoniae capsulée RO3O ;
3 - Pseudomonas aeruginosa CNCM A22 ;
4 - Streptococcus faecalis CNCM 5855 ;
5 - Staphylococcus aureus CNCM 53154.

La deuxième souche est entretenue sur milieu Worgel Fergusson, les autres sur Tryptic Soy Agar-Difco (TSA), commercialisé par Difco.

Après 24 h de culture à 37° C, on récolte la pousse microbienne à l'aide de billes de verre et de 1O ml de diluant contenant 1 g de tryptone et 8,5 g de chlorure de sodium dans 1 OOO ml d'eau distillée. On agite la suspension formée et on mesure au spectrophotomètre le pourcentage de transmission de la lumière à 62O nm :

- Souche 1 : 70 % ;
- Souche 2 : 80 % ;
- Souche 3 : 70 % ;
- Souche 4 : 60 % ;
- Souche 5 : 60 %.

L'inoculum bactérien correspond à une dilution au 1/2O de cette suspension bactérienne.

Des plaques comportant des cupules reçoivent différentes dilutions du produit à étudier. Ces dilutions du produit à étudier sont mises en contact avec les différentes suspensions bactériennes à l'aide d'un inoculateur à site multiple. Après 2O min de contact, des parties aliquotes sont transférées à l'aide de cet inoculateur à la surface d'un milieu gélosé (TSA) placé dans des boîtes de Petri, contenant un neutralisant de l'activité, à savoir 20 g de lubrol W, 25 g de Tween 8O et 2,5 g de thiosulfate de sodium dans 1 OOO ml de TSA (Difco). Un témoin de l'efficacité du neutralisant est réalisé pour chaque produit étudié en déposant à la surface du milieu de culture une partie aliquote de la dilution du produit à étudier. Après séchage, l'inoculum correspondant est déposé au même endroit. Un témoin inoculum est réalisé sur milieu gélosé avec et sans neutralisant. La lecture se fait après 48 h d'incubation à 37° C.

Les résultats sont rassemblés dans le tableau I ci-dessous.

TABLEAU I

| CONCENTRATION MINIMALE BACTERICIDE (CMB) en µq/ml | | | | | |
|---|---|---|---|---|---|
| n° du produit | Souches bactériennes | | | | |
| | 1 | 2 | 3 | 4 | 5 |
| SR 42 643 A | 5000 | 5000 | 1000 | 2000 | 1000 |
| SR 42 989 A | 1500 | 1500 | 1500 | 2000 | 1500 |
| SR 42 994 A | 1000 | 800 | 1000 | 1000 | 1000 |
| SR 43 063 A | 2000 | 1000 | 1000 | 2000 | 1000 |
| SR 43 077 A | 600 | 600 | 800 | 600 | 600 |
| SR 43 087 A | 1500 | 1000 | 800 | 2000 | 800 |
| SR 43 121 A | 1500 | 1500 | 1500 | 800 | 800 |
| SR 43 154 A | 2000 | 1500 | 800 | 1500 | 1000 |
| SR 43 155 A | 500 | 500 | 200 | 500 | 500 |
| SR 43 157 A | 200 | 200 | 100 | 200 | 200 |
| SR 43 245 A | 1500 | 1000 | 800 | 1500 | 1000 |
| SR 43 247 A | 300 | 300 | 300 | 300 | 500 |
| SR 43 270 A | 1000 | 1000 | 1000 | 2000 | 1000 |
| SR 43 290 A | 1500 | 800 | 800 | 4000 | 800 |
| SR 43 292 A | 300 | 300 | 300 | 300 | 300 |
| SR 43 293 A | 200 | 200 | 200 | 200 | 200 |
| SR 43 382 A | 5000 | 2000 | 2000 | 5000 | 2000 |
| SR 43 383 A | 100 | 100 | 100 | 100 | 100 |
| SR 43 703 A | 600 | 600 | 400 | 800 | 1000 |
| SR 43 705 A | 500 | 500 | 500 | 500 | 500 |
| SR 43 727 A | 20 | 50 | 50 | 20 | 20 |
| SR 43 802 A | 1000 | 1000 | 1000 | 2000 | 5000 |
| SR 43 803 A | 400 | 400 | 500 | 400 | 400 |
| SR 43 826 A | 50 | 50 | 50 | 50 | 50 |
| SR 43 940 A | 50 | 50 | 50 | 50 | 50 |
| SR 43 941 A | 50 | 50 | 50 | 50 | 50 |
| SR 43 969 A | 50 | 100 | 500 | 100 | 50 |
| SR 43 971 A | 500 | 500 | 500 | 500 | 500 |
| SR 44 027 A | 50 | 50 | 100 | 100 | 200 |
| SR 44 029 A | 10 | 50 | 50 | 50 | 50 |
| SR 44 226 A | 1000 | 1000 | 500 | 1000 | 2000 |
| SR 44 227 A | 100 | 300 | 500 | 100 | 500 |

Les résultats montrent que les produits selon l'invention présentent un large spectre d'activité sur les souches bactériennes testées. Cette activité bactéricide s'exprime dans un temps court (20 min).

A titre de comparaison, on a mesuré dans les mêmes conditions la concentration minimale bactéricide (CMB) en µg/ml des composés suivants :

SR 43 970 A

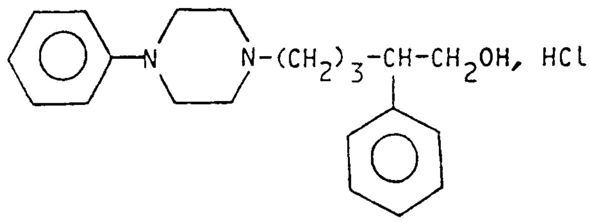

SR 43 291 A

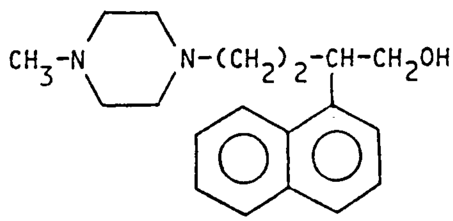

| CONCENTRATION MINIMALE BACTERICIDE (CMB) en µg/ml | | | | | |
|---|---|---|---|---|---|
| n° du produit | Souches bactériennes | | | | |
| | 1 | 2 | 3 | 4 | 5 |
| SR 43 970 A<br>SR 43 291 A | 5000<br>5000 | 5000<br>3000 | 2000<br>1000 | 10000<br>>10000 | 5000<br>8000 |

Ces produits ont une activité bactéricide nettement inférieure aux composés selon l'invention.

L'activité antifongique des produits selon l'invention a également été déterminée en utilisant la méthode décrite précédemment.

Les souches fongiques choisies pour l'étude sont :

1 - Candica albicans CNCM 118O ;
2 - Candida tropicalis 3834 ;
3 - Candida parakruzeï 3918 ;
4 - Torulopsis glabrate O72O23.

Elles sont entretenues sur milieu gélosé de Sabouraud Dextrose Agar, commercialisé par Difco, la technique est identique à celle décrite pour l'étude de l'activité antibactérienne. Après 48 h de culture à 37°C, on récolte la pousse microbienne à l'aide de billes de verre et de 5 ml de diluant contenant 1 g de tryptone et 8,5 g de chlorure de sodium dans 1 OOO ml d'eau distillée ; 5 ml du diluant sont ensuite rajoutés. Cette suspension donne au spectromètre un pourcentage de transmission de la lumière à 62O nm de 2 à 3 %. Une dilution au 1/1OO de cette suspension, observée entre lame et lamelle à l'objectif 4O d'un microscope, doit montrer 1O cellules par champ, ce qui correspond à 1 OOO OOO levures par ml.

Les résultats sont rassemblés dans le tableau II ci-dessous.

TABLEAU II

| CONCENTRATION MINIMALE FONGICIDE (CMF) en µg/ml | | | | |
|---|---|---|---|---|
| n° de produit | Souches de levures | | | |
| | 1 | 2 | 3 | 4 |
| SR 42 994 A | 500 | 500 | 500 | 5000 |
| SR 43 077 A | 1000 | 1000 | 1000 | 5000 |
| SR 43 087 A | 1000 | 1000 | 1000 | 5000 |
| SR 43 155 A | 500 | 500 | 500 | 2000 |
| SR 43 157 A | 300 | 300 | 300 | 1000 |
| SR 43 247 A | 1000 | 500 | 1000 | 2000 |
| SR 43 270 A | 500 | 1000 | 1000 | 5000 |
| SR 43 292 A | 1000 | 1000 | 1000 | 2000 |
| SR 43 293 A | 500 | 200 | 500 | 1000 |
| SR 43 382 A | 5000 | 1000 | 2000 | 10000 |
| SR 43 383 A | 100 | 100 | 100 | 500 |
| SR 43 803 A | 1000 | 500 | 500 | 2000 |
| SR 43 826 A | 250 | 50 | 50 | 250 |
| SR 43 941 A | 100 | 100 | 250 | 250 |
| SR 43 969 A | 50 | 50 | 100 | 500 |
| SR 44 027 A | 250 | 250 | 500 | 250 |
| SR 44 029 A | 50 | 50 | 50 | 100 |

Ces résultats montrent que les produits selon l'invention possèdent une activité antifongique intéressante et rapide.

La tolérance des produits selon l'invention a été étudiée chez le cobaye. Les animaux sont tondus de part et d'autre de la ligne médiane du dos, la tonte est entretenue tous les 2 jours. Des lots de 6 animaux reçoivent sur la zone tondue 0,2 ml d'une solution aqueuse ou alcoolique du produit selon l'invention. Lorsque les produits sont en solution alcoolique, un lot témoin d'animaux reçoit l'alcool sur un côté.

Pour l'étude de la tolérance cutanée préliminaire, le traitement est appliqué 1 fois par jour, 6 jours sur 7, durant 3 semaines. Les observations sur le plan cutané portent sur la présence d'érythème, d'éruption cutanée ou d'hyperkératose dont l'intensité est graduée selon un barème déterminé.

L'essai de sensibilisation cutanée est réalisé sur les mêmes animaux après deux semaines de repos. Le traitement dure une semaine, il est identique au précédent. L'évaluation se fait sur les mêmes critères et d'après le même barème que celui utilisé pour la tolérance locale.

On a également recherché si les produits selon l'invention présentent un effet phototoxique ou photoallergique chez le cobaye. La technique mise en oeuvre est celle de J. UNKOVIC, G. MAZUE et J. GIRARD, dans Sciences et Techniques de l'Animal de Laboratoire, 1983, 8 (3), 149-16O. C'est une adaptation des techniques décrites par L.C. HARBER et al., Arch. Dermatol., 1967, 96, 646-656 et L.J. VINSON et al., Arch. Dermatol., 1966, 17, 123-13O.

Aucun des produits étudiés, à savoir SR 42 643 A, SR 43 O77 A, SR 43 157 A, SR 43 27O A, SR 43 292 A, SR 43 293 A, SR 43 383 A, SR 43 727 A, n'a montré de mauvaise tolérance, d'effet sensibilisant ni d'effet phototoxique ou photoallergique chez le cobaye.

Une évaluation de la toxicité aiguë par Voie orale a été réalisée chez la souris. Cette étude a été effectuée sur des souris mâles de souche CD1 provenant de l'élevage Charles River. Chaque lot était composé de 5 animaux d'un poids corporel variant de 24 à 3O g entretenus dans une même cage. Les animaux étaient conservés à jeun pendant 6 h avant le traitement. Pour chaque étude, le produit, mis en suspension dans une solution de gomme arabique à 1O %, a été administré par gavage à l'aide d'une sonde oesophagienne. La nourriture était de nouveau distribuée aux animaux 4 h après le gavage et les animaux étaient gardés en observation pendant une période de 14 jours après l'administration. Pendant cette période, on note la mortalité dans chacun des lots mis en expérience et, lorsque cela est possible, on détermine la dose létale 5O (DL 5O) en utilisant la méthode de J.T. LITCHFIELD et F. WILCOXON, J. Pharmacol. 1949, 95, 99-113.

Les résultats sont exprimés en mg de substance essayée par kg de poids corporel. Ils sont rassemblés dans le tableau III ci-dessous.

TABLEAU III

| TOXICITE AIGUE PER OS CHEZ LA SOURIS (mg/kg) | |
|---|---|
| N° du produit | DL 50 |
| SR 42 643 A | 400 |
| SR 42 989 A | 300-400 |
| SR 42 994 A | 750-1000 |
| SR 43 063 A | 300-400 |
| SR 43 077 A | 300-400 |
| SR 43 121 A | 300-400 |
| SR 43 155 A | inférieur à 500 |
| SR 43 292 A | 500-750 |
| SR 43 293 A | inférieur ou égal à 250 |
| SR 43 383 A | 1180 |
| SR 43 727 A | 580 |
| SR 44 027 A | 1000-1200 |

Les exemples suivants illustrent l'invention sans toutefois la limiter.

Exemple 1

Chlorhydrate de N,N-diéthylamino-4 (naphtyl-1)-2 butanol. SR 42 989 A.

1) N,N-diéthylamino-4 (naphtyl-1)-2 butyronitrile.

On ajoute par petites portions 7 g d'amidure de sodium à 27,11 g de naphtyl-1 acétonitrile dans 210 ml de benzène anhydre. On chauffe 2 h au reflux et on ajoute 22 g de chloro-2 diéthylamino-éthane puis on continue à chauffer au reflux pendant 2 h 30 min. On refroidit, ajoute 200 ml d'eau. Après décantation, la phase organique est extraite par HCl à 10 %. La phase aqueuse est lavée à l'éther, neutralisée par NaOH à 10 %, puis extraite à l'éther. Après évaporation des solvants, on obtient une huile rouge qui est distillée sous vide.

Eb = 158-160 ° C sous 0,04 mmHg soit 0,053 mbar ;
P = 27 g ;
Rendement = 63 %.

2) Ester éthylique de l'acide N,N-diéthylamino-4-(naphtyl-1)-2 butyrique.

16 g du produit obtenu précédemment sont chauffés au reflux pendant 2 h dans 66 ml d'un mélange équivolumique d'acide sulfurique, acide acétique et eau. On refroidit, dilue à l'eau et lave à l'éther. La phase aqueuse est neutralisée par de la soude à 3O % et lavée à l'éther. Puis la phase aqueuse est à nouveau acidifiée par l'acide chlorhydrique concentré puis évaporée à sec, on extrait le résidu à l'éthanol chaud. Après avoir évaporé l'éthanol, on reprend le résidu par 1OO ml d'éthanol auxquels on ajoute quelques gouttes d'acide sulfurique concentré, puis on chauffe une nuit à reflux. Après refroidissement, on évapore, reprend par de l'eau, neutralise par addition de bicarbonate de sodium puis on extrait à l'éther, lave à l'eau et sèche sur sulfate de magnésium. On obtient ainsi 1O g d'une huile orange.

Rendement brut = 63,85 %.

3) SR 42 989 A

10 g du produit obtenu précédemment sont ajoutés goutte à goutte à 15,5 g de Vitride®(hydrure de sodium bis(méthoxy-2 éthoxy) aluminium) à 7O % dans du toluène. Après 2 h à température ambiante, on verse sur l'eau et sèche sur sulfate de magnésium. Puis on évapore, reprend à l'éther éthylique sec et on ajoute goutte à goutte de l'éther éthylique chlorhydrique. On évapore à nouveau, reprend par de l'alcool isopropylique et ajoute goutte à goutte de l'éther éthylique anhydre. Le précipité formé est filtré, lavé à l'éther éthylique anhydre et séché. On obtient 6 g du produit attendu qui est recristallisé dans l'éther.

Rendement = 65 % ;

Fc = 98-100 ° C.

Exemple 2

Chlorhydrate de pipéridino-8 (naphtyl-1)-2 octanol. SR 43 157 A.

Les 3 premières étapes permettent de préparer le chloro-1 pipéridino-6 hexane.

1) Pipéridino-6 oxo-6 hexanoate d'éthyle.

On chauffe au reflux pendant 1 h 3O min 52,2 g de monoester éthylique de l'acide adipique dans 3O ml de chlorure de thionyle puis on évapore sous vide l'excés de chlorure de thionyle et reprend par 1OO $cm^3$ d'éther anhydre. On ajoute goutte à goutte à O ° C, 58,8 g de pipéridine dans 1OO ml d'éther et laisse revenir à température ambiante en agitant pendant 1 h. On verse sur de l'eau, décante la phase organique, lave 2 fois à l'eau puis avec une solution de carbonate de sodium à 1O %, sèche sur sulfate de magnésium et évapore le solvant pour obtenir 4O g d'une huile brune que l'on distille sous vide.

Eb = 132-136 ° C sous O,O25 mmHg, soit O,O33 mbar ;

P = 35 g ;

Rendement = 48 %.

2) Pipéridino-6 hexanol.

On ajoute goutte à goutte 35 g du produit obtenu précédemment à 72,8 g de Vitride à 7O % dans le toluène puis on laisse une nuit à température ordinaire. On verse ensuite sur de la glace, extrait à l'éther, lave à l'eau, sèche sur sulfate de magnésium et évapore sous vide.

On obtient 24 g de liquide incolore.

Rendement brut : 8O %.

3) Chloro-1 pipéridino-6 hexane.

On ajoute goutte à goutte 16 g de chlorure de thionyle à 24 g du produit obtenu précédemment dissous dans 75 ml de chloroforme. Après 4 h de chauffage à reflux, on évapore le solvant, reprend à l'eau et neutralise la solution par de la soude à 1O %, on extrait à l'éther, lave à l'eau, sèche sur sulfate de magnésium et évapore le solvant.

P = 22 g ;

Rendement brut = 91,6 %.

4) Pipéridino-8 (naphtyl-1)-2 octanenitrile.

On ajoute par petites portions 3,9 g d'amidure de sodium à 16,7 g de naphtyl-1 acétonitrile dissous dans 2OO ml d'éther anhydre. Après 2 h de chauffage à reflux, on ajoute 2O,5 g de pipéridino-6 chloro-1 hexane et on chauffe à nouveau au reflux pendant 5 h. En utilisant les méthodes habituelles, on isole ensuite 3O g de produit.

Rendement = 89,7 %.

5) Pipéridino-8 (naphtyl-1)-2 octanoate d'éthyle.

30 g du produit obtenu précédemment sont chauffés à reflux pendant 2 h dans 15O ml d'un mélange équivolumique d'acide sulfurique, acide acétique et eau. Après refroidissement, on verse sur de l'eau et lave à l'éther, puis la phase aqueuse est rendue basique par addition de soude à 3O % et on décante l'huile formée. La phase organique est lavée à l'éther puis l'huile est acidifiéê à pH = 1 par addition d'acide chlorhydrique concentré, on reprend par de l'éthanol à 1OO %, ajoute quelques gouttes d'acide sulfurique concentré et chauffe une nuit au reflux. On évapore l'alcool sous vide, reprend par de l'eau, neutralise par addition de carbonate acide de sodium, extrait à l'éther puis on lave jusqu'à neutralité et évapore le solvant sous vide. On obtient 23 g d'un produit huileux.

Rendement = 67,6 %.

6) SR 43 157 A.

A partir de 7,5 g du produit obtenu à l'étape précédente et en utilisant la méthode décrite à l'exemple 1, étape 3, on obtient le produit attendu qui est recristallisé dans le mélange méthanol 100-éther (1/1 v/v).

P = 2,8 g.

Fc = 125-129 ° C.

Rendement = 38 %.

Exemple 3

Chlorhydrate d'éthyl-2 (naphtyl-1)-2 pipéridino-4 butanol. SR 43 245 A.

1) (Naphtyl-1)-2 pipéridino-4 butyronitrile.

On ajoute par petites portions 8,2 g d'amidure de sodium sur une solution de 33,4 g de naphtyl-1 acétonitrile dans 450 ml d'éther anhydre. On chauffe 2 h au reflux et on ajoute 29,5 g de pipéridino-2 chloro-1 éthane puis on chauffe 5 h au reflux. On refroidit, verse dans l'eau, puis la phase organique est extraite 3 fois avec 600 ml d'acide chlorhydrique à 10 %. La phase aqueuse est lavée à l'éther, neutralisée par de la soude à 30 %. On extrait l'huile qui décante, lave par de l'eau saturée en chlorure de sodium, sèche sur sulfate de magnésium. Après évaporation du solvant, l'huile obtenue est distillée sous pression réduite (pompe à palettes).

P = 41,5 g ;

Eb = 172-176 ° C sous 0,025 mmHg soit 0,033 mbar.

2) Ethyl-2 (naphtyl-1)-2 pipéridino-4 butyronitrile.

On ajoute par petites portions 6,5 g de $NaNH_2$ sur une solution de 41,5 g du produit obtenu précédemment dans 300 ml d'éther anhydre. On chauffe 2 h au reflux, puis ajoute goutte à goutte 17,44 g de bromure d'éthyle. Après 5 h de chauffage au reflux, on verse sur l'eau, extrait la phase organique par de l'acide chlorhydrique à 10 %. La phase aqueuse est lavée à l'éther, neutralisée par de la soude à 30 %. On extrait l'huile qui décante par de l'éther, lave par de l'eau saturée en chlorure de sodium, sèche sur sulfate de magnésium et évapore à sec.

P = 44 g.

3) Chlorhydrate d'éthyl-2 (naphtyl-1)-2 pipéridino-4-butanamide.

A 44 g du produit obtenu précédemment, on ajoute 120 ml d'un mélange équivolumique d'eau, acide sulfurique concentré et acide acétique. Après 24 h de chauffage à reflux, on verse sur de l'eau, neutralise par de la soude à 30 %. L'huile qui décante est extraite à l'éther, lavée à l'eau, séchée sur sulfate de magnésium et évaporée à sec. Le résidu est repris par de l'éther et on ajoute goutte à goutte de l'éther chlorhydrique. On évapore le solvant à froid, dissout le produit dans 200 ml d'éthanol 100 %, puis précipite à l'éther, filtre, lave à l'éther et sèche sous vide.

P = 40 g.

4) Acide éthyl-2 (naohtyl-1)-2 pipéridino-4 butanoïque.

On fait buller de l'acide chlorhydrique gazeux pendant 1 h 30 min sur une solution de 20 g du produit obtenu précédemment dans 10 ml d'acide acétique, en maintenant la température inférieure à 10 ° C. On ajoute en 1 h, à 0 ° C, 20 ml de nitrite d'isopentyle. On chauffe 7 h à 100 ° C puis laisse réagir 2 jours à température ambiante. On évapore à sec, reprend à l'éther, évapore l'éther, dissout le précipité dans 100 ml d'éthanol 100 ; le produit précipite dans l'éther, on le dissout dans l'eau, neutralise à la soude, lave à l'éther et acidifie avec de l'acide chlorhydrique. On évapore à sec, reprend par de l'eau, le produit précipite à pH 1,3. On filtre, lave à l'eau froide et sèche sous vide avec de l'anhydride phosphorique.

P = 10 g ;

Rendement = 66 %.

5) SR 43 245 A.

On ajoute goutte à goutte 10 g du produit obtenu précédemment sur 25 ml de itride dans 100 ml de toluène. On chauffe 24 h à 80 ° C, verse dans l'eau, sèche sur sulfate de magnésium. On évapore à sec,

reprend à l'éther et ajoute goutte à goutte de l'éther chlorhydrique. On évapore le solvant à froid, le précipité est dissous dans ml d'éthanol 1OO puis précipité dans l'éther ; on filtre, lave à l'éther et sèche sous vide. Après recristallisation dans le mélange éthanol 1OO-éther (1/1 v/v), on obtient le produit attendu.

P = 2,5 g.

Rendement = 25 %.

Fc = 162-165 ° C.

Exemple 4

Chlorhydrate de (benzyl-4 pipéridino)-4 (dichloro-3,4 phényl)-2 butanol. SR 43 969 A.

1) (Benzyl-4 pipéridino)-4 (dichloro-3,4 phényl)-2 butyronitrile.

On ajoute, par petites portions, 1,95 g d'amidure de sodium à une solution éthérée de 9,3 g de dichloro-3,4 phénylacétonitrile. On chauffe 2 h au reflux, on ajoute 11,8 g de (benzyl-4 pipéridino)-2 chloro-1 éthane et on chauffe à nouveau 5 h au reflux. On refroidit, ajoute 2OO ml d'eau, décante la phase organique, lave à l'eau et extrait par une solution d'acide chlorhydrique à 1O%. La phase aqueuse est ensuite neutralisée par la soude, extraite à l'éther, lavée à l'eau, séchée sur sulfate de magnésium et évaporée. On obtient ainsi 13 g de produit huileux.

Rendement brut : 69 %.

2) (Benzyl-4 pipéridino)-4 (dichloro-3,4 phényl)-2 butanoate de méthyle.

On chauffe à reflux pendant 2 h, 13 g du produit obtenu à l'étape précédente dans 6O ml d'une solution acide sulfurique/eau/acide acétique (1/1/1 en volume). On refroidit, verse sur l'eau et lave à l'éther. La phase aqueuse est lavée par de la soude à 3O%, puis on décante l'huile formée et lave trois fois à l'éther. On acidifie l'huile à pH1 par de l'acide chlorhydrique à 35 % et évapore à sec. On reprend le résidu avec du méthanol additionné de quelques gouttes d'acide sulfurique et on chauffe au reflux pendant 4 h. On refroidit, évapore sous vide, reprend à l'eau, neutralise au bicarbonate de sodium, extrait à l'éther, lave à l'eau, sèche sur sulfate de magnésium, puis à l'évaporateur rotatif. On obtient 9 g de produit huileux.

Rendement brut : 63 %.

3) SR 43 969 A.

9 g de produit obtenu à l'étape précédente sont ajoutés à 6,45 g de Vitride® en solution dans 5O ml de toluène. On agite min à température ambiante. On verse sur l'eau, décante la phase organique, lave à l'eau, sèche sur sulfate de magnésium et évapore. Le résidu est repris à l'éther et on précipite par addition d'éther chlorhydrique. On évapore l'éther, reprend le résidu par un minimum d'éthanol 1OO, puis on additionne de l'éther éthylique jusqu'à formation d'un trouble, puis cristallisation. Après recristallisation dans un mélange éther-éthanol (1/1 v/v), on obtient 7 g du produit attendu.

Rendement : 81,7 % ;

Fc : 167,5% ° C.

En utilisant les mêmes méthodes que précédemment, on a préparé les produits selon l'inVention, qui sont décrits dans les tableaux IV et V ci-dessous. Ils sont caractérisés par leur point de fusion (Fc) après recristallisation dans un solvant. Les solvants de recristallisation (solvant) sont utilisés purs ou en mélange équivolumique. La signification des abréviations est la suivante :

- Alcool méthylique : MeOH ;
- Alcool éthylique : EtOH ;
- Alcool isopropylique : iPrOH ;
- Ether éthylique : $Et_2O$
- Ether isopropylique : $(iPr)_2O$
- Dichlorométhane : DCM.

Tableau IV

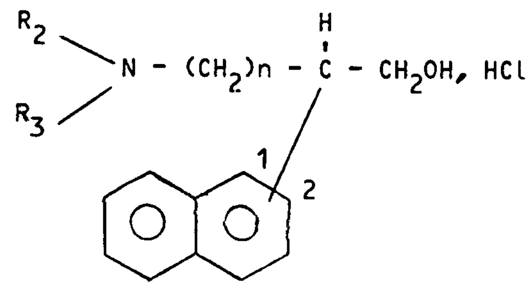

| N° SR | n | N-$R_2$ $R_3$ | Fc °C | Solvant |
|---|---|---|---|---|
| 42 643 A | 2 | diméthylamino | 163 | EtOH / $(iPr)_2O$ |
| 42 994 A | 3 | diméthylamino | 120 - 125 | iPrOH / $Et_2$ O |
| 43 063 A | 2 | pyrrolidino | 127 - 132 | iPrOH / $Et_2$ O |
| 43 077 A | 2 | pipéridino | 168 - 172 | iPrOH / $Et_2$ O |
| 43 087 A | 3 | pipéridino | 152 - 155 | EtOH |
| 43 121 A | 2 | $N(CH(CH_3)_2)_2$ | 160 - 165 | EtOH / $Et_2$ O |
| 43 154 A* | 2 | pipéridino | 183 - 185 | EtOH |
| 43 155 A | 2 | hexaméthylèneimino | 169 - 175 | EtOH |
| 43 247 A | 2 | tétrahydro-1,2,3,4 isoquinol-2 yl | 173 - 176 | EtOH / $Et_2$ O |
| 43 270 A* | 2 | hexaméthylèneimino | 172 - 175 | EtOH / $Et_2$ O |
| 43 290 A | 3 | méthyl-4 pipéridino | 137 - 142 | EtOH |
| 43 292 A | 2 | benzyl, isopropylamino | 205 - 209 | MeOH / $Et_2$ O |
| 43 293 A | 6 | méthyl-4 pipéridino | 152 - 154 | MeOH / $Et_2$ O |
| 43 382 A | 2 | méthyl-4 pipéridino | 200 - 202 | EtOH / $Et_2$ O |
| 43 383 A | 2 | benzyl-4 pipéridino | 185 - 186 | EtOH / $Et_2$ O |
| 43 727 A** | 6 | diméthylamino | RMN | |
| 43 826 A** | 9 | diéthylamino | RMN | |
| 43 940 A | 6 | diéthylamino | 86 - 89 | $Et_2O$ |
| 43 941 A | 5 | benzyl-4 pipéridino | 85 | DCM / $Et_2O$ |
| 44 227 A | 2 | dicyclohexylamino | 110 - 114 | $(iPr)_2O$ |

* Les composés SR 43 154 A et SR 43 270 A sont substitués en 2 sur le naphtalène. Tous les autres composés de ce tableau sont substitués en 1 sur le naphtalène.

** Les SR 43 727 A et SR 43 826 A sont obtenus sous forme d'une huile et caractérisés par leur spectre de résonance magnétique nucléaire (RMN) :

<u>SR 43 727 A (Spectre enregistré à 60 MHz)</u> :

| | | |
|---|---|---|
| 10 protons entre 0,7 et 1,9 ppm | ; massif | ; $-\underline{(CH_2)_5}-CH-CH_2OH$ |
| 8 protons entre 2,3 et 3 ppm | ; massif | ; $(\underline{CH_3})_2N-\underline{CH_2}-$ |
| 3 protons entre 3,4 et 3,8 ppm | ; massif | ; $-C\underline{H}-\underline{CH_2}-OH$ |
| 7 protons entre 7,1 et 8,3 ppm | ; massif | ; H aromatique du naphtalène |
| 1 proton à 10,4 ppm | ; massif | ; $O\underline{H}$ |

<u>SR 43 826 A (Spectre du produit sous forme de base, enregistré à 250 MHz)</u> :

| | | |
|---|---|---|
| 14 protons entre 1 et 1,35 ppm | ; multiplet | ; $\underline{(CH_2)_7}-CH_2-CH-CH_2OH$ |
| 6 protons entre 0,73 et 0,96 ppm | ; triplet | ; $(\underline{CH_3}-CH_2)_2N$ |
| 2 protons entre 1,5 et 2 ppm | ; multiplet | ; $-\underline{CH_2}-CH-CH_2-OH$ |
| 2 protons à 2,2 ppm | ; triplet | ; $N-\underline{CH_2}-$ |
| 4 protons entre 2,3 et 2,4 ppm | ; quadruplet | ; $(CH_3-\underline{CH_2})_2N$ |
| 3 protons entre 3,4 et 3,7 ppm | ; multiplet | ; $C\underline{H}-\underline{CH_2}-OH$ |
| 1 proton à 4,6 ppm | ; singulet | ; $O\underline{H}$ |
| 7 protons entre 7,3 et 8,2 ppm | ; massif | ; H aromatiques du naphtalène. |

TABLEAU V

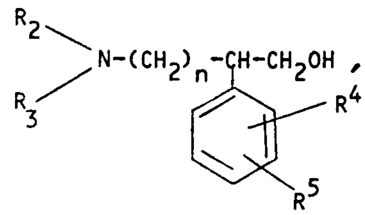 , HCl

| n° SR | n | N $R_2$ $R_3$ | $R_4$, $R_5$ | F.°C | Solvant |
|---|---|---|---|---|---|
| 43 110 A | 2 | pipéridino | H, H | 154-158 | iPrOH/$Et_2O$ |
| 43 111 A | 3 | pipéridino | H, H | 120-125 | iPrOH/$Et_2O$ |
| 43 120 A | 2 | pyrrolidino | H, H | 115-117 | EtOH/$Et_2O$ |
| 43 408 A | 2 | pipéridino | 4 F, H | 165-167 | EtOH/$Et_2O$ |
| 43 409 A | 2 | pipéridino | 2 Cl, H | 183-185 | EtOH/$Et_2O$ |
| 43 703 A | 2 | hexaméthylène-imino | 2 Cl, 6 Cl | 135-147 | EtOH/$Et_2O$ |
| 43 705 A | 2 | benzyl-4 pipéridino | H, H | 170-172 | EtOH/$Et_2O$ |
| 43 802 A | 2 | hexaméthylèneimino | 2 Cl, 4 Cl | 162-165 | iPrOH/$Et_2O$ |
| 43 803 A | 2 | hexaméthylèneimino | 3 Cl, 4 Cl | 155 | iPr OH |
| 43 971 A | 2 | benzyl-4 pipéridino | 3 Cl, 4 Cl | 176-179 | EtOH/$Et_2O$ |
| 44 027 A | 2 | " | 2 Cl, 4 Cl | 160-162 | EtOH/$Et_2O$ |
| 44 028 A | 2 | " | 2 Cl, 6 Cl | 160-162 | EtOH/$Et_2O$ |
| 44 029 A | 2 | " | 4 $C_6H_5$, H | 211-213 | EtOH/$Et_2O$ |
| 44 226 A | 2 | " | 2 $CH_3$, H | 172-175 | EtOH/$Et_2O$ |
| 44 245 A** | 6 | diéthylamino | 3 Cl, 4 Cl | RMN | |
| 44 246 A | 2 | benzyl-4 pipéridino | 2 Cl, H | 178-180 | iPrOH |

** Le SR 44 245 A obtenu sous forme d'huile est caractérisé par son spectre RMN enregistré à 250 MHz.

SR 44 245 A :

16 protons entre 0,96 et 1,72 ppm; massif ; $N(CH_2C\underline{H_3})_2$, $(C\underline{H_2})_4$ et $C\underline{H_2}$-CH-

1 proton entre 2,55 et 2,70 ppm ; massif ; $CH_2$-C$\underline{H}$-

6 protons entre 2,8 et 3,10 ppm ; massif ; N-$(C\underline{H_2}CH_3)_2$ et -$C\underline{H_2}$N<

2 protons entre 3,4 et 3,5 ppm ; massif ; $C\underline{H_2}OH$

1 proton entre 4,6 et 4,7 ppm ; triplet ; O$\underline{H}$

1 proton entre 7,12 et 7,19 ppm ; doublet ; H aromatique

1 proton à 7,4 ppm ; singulet ; H aromatique

1 proton entre 7,45 et 7,50 ppm ; doublet ; H aromatique.

Différentes formulations galéniques des produits selon l'invention peuvent être préparées selon l'application choisie.

Exemple 5

| Préparation antiseptique liquide détergente moussante. | |
|---|---|
| SR 43 383 A | 0,2 g |
| Alkyldiméthylcarboxyméthylamine (solution à 30 %) | 15 g |
| Tétracémate disodique | 0,1 g |
| Propylèneglycol | 20 g |
| Hydroxyde de sodium qsp pH 5,8 | |
| Eau purifiée qsp | 100 g |

Exemple 6

| Préparation antiseptique liquide détergente moussante. | |
|---|---|
| SR 43 293 A | 0,5 g |
| Paraffine sulfonate de sodium | 15 g |
| Hydroxyde de sodium ou acide lactique qsp pH 5,2 | |
| Eau purifiée qsp | 100 g |

Exemple 7

| Soluté antiseptique alcoolique. | |
|---|---|
| SR 43 247 A | 0,5 g |
| Alkyldiméthylcarboxyméthylamine (solution à 30 %) | 0,5 g |
| Condensat d'oxyde d'éthylène et de propylèneglycol L 62 | 1 g |
| Acide lactique ou hydroxyde de sodium qsp pH 6,5 | |
| Alcool éthylique à 70° qsp | 100 g |

Exemple 8

Un produit selon l'invention peut être utilisé comme conservateur dans un shampooing.

| | |
|---|---|
| Palmitate de potassium et d'acides aminés | 20 g |
| Alkylsulfates de sodium | 2 g |
| Diéthanolamide de coprah | 5 g |
| Acétate de linalyle | 0,200 g |
| SR 43 157 A | 0,100 g |
| Hydroxyde de sodium qsp pH 7 | |
| Eau purifiée qsp | 100 g |

Exemple 9

Un produit selon l'invention peut être utilisé comme conservateur dans une crème émulsion.

| | | |
|---|---|---|
| Huile de vaseline | | 6 g |
| Mélange d'alcool cétostéarylique et d'alcool cétostéarylique oxyéthylené | | 9 g |
| Phosphate monosodique anhydre | | 0,300 g |
| Tétracémate disodique | | 0,010 g |
| Vaseline | | 15 g |
| SR 43 292 A | | 0,100 g |
| Acide phosphorique | qsp pH 4,5 | |
| Eau purifiée | qsp | 100 g |

Exemple 10

Le produit selon l'invention peut être utilisé comme conservateur dans une crème pour utilisation cosmétologique.

| | | |
|---|---|---|
| Collagène | | 0,500 g |
| Carboxypolyméthylène 934 | | 0,400 g |
| Lanoline hydrogénée | | 4 g |
| Perhydrosqualène | | 20 g |
| Monopalmitate de sorbitol polyoxyéthylené | | 2 g |
| SR 43 293 A | | 0,150 g |
| Acide lactique ou hydroxyde de sodium | qsp pH 6,5 | |
| Eau purifiée | qsp | 100 g |

Exemple 11

Conservateur dans une crème pour utilisation cosmétologique :

| | |
|---|---|
| Collagène | 0,500 g |
| Carboxypolyméthylène 934 | 0,400 g |
| Lanoline hydrogénée | 4 g |
| Perhydrosqualène | 20 g |
| Monopalmitate de sorbitol polyoxy-éthylené | 2 g |
| SR 43 940 A | 0,150 g |
| Acide lactique ou hydroxyde de sodium | qsp pH 6,5 |
| Eau purifiée | qsp 100 g |

Exemple 12

| Conservateur dans une huile antisolaire : | |
|---|---|
| Huile minérale 65/75 | 68 g |
| Huile de castor | 8 g |
| Huile de sésame | 20 g |
| Alcool isopropylique | 2 g |
| Eusolex 6300 | 1,5 g |
| Parfum | 0,4 g |
| SR 43 292 A | 0,100 g |

Exemple 13

| Conservateur dans une huile antisolaire : | |
|---|---|
| Huile minérale 65/75 | 68 g |
| Huile de castor | 8 g |
| Huile de sésame | 20 g |
| Alcool isopropylique | 2 g |
| Eusolex 6300 | 1,5 g |
| Parfum | 0,4 g |
| SR 44 029 A | 0,100 9 |

Exemple 14

Conservateur pour jus de fruits ou confiture :

SR 43 383 A micronisé 0,02 %.

Exemple 15

Désinfectant pour surfaces inertes :

| | | |
|---|---|---|
| SR 43 157 A | | 2 g |
| Dodécyldiméthylcarboxydiméthylamine | | 20 g |
| Tétracémate disodique | | 2 g |
| Acide lactique | qsp pH 3,5 | |
| Eau purifiée | qsp | 100 g |

Exemple 16

Désinfectant pour surfaces inertes :

| | | |
|---|---|---|
| SR 44 027 A | | 2 g |
| Dodécyldiméthylcarboxydiméthylamine | | 20 g |
| Tétracémate disodique | | 2 g |
| Acide lactique | qsp pH 3,5 | |
| Eau purifiée | qsp | 100 g |

Exemple 17

Protection des agents viscosifiants utilisés en récupération améliorée des hydrocarbures :

- Solution aqueuse à 1000 ppm de polymère synthétique (polyacrylamide) ou de biopolymère (xanthane)
- SR 43 727 A 1000 ppm

Une telle concentration en biocide protège la solution aqueuse de polymère ou de biopolymère des risques de biodégradation dans le réservoir pendant la phase de récupération des hydrocarbures.

**Revendications**

**Revendications pour les Etats contractants suivants : BE, CH, DE, FR, GB, IT, LI, LU, NL, SE**

**1.** Dérivés aromatiques de formule

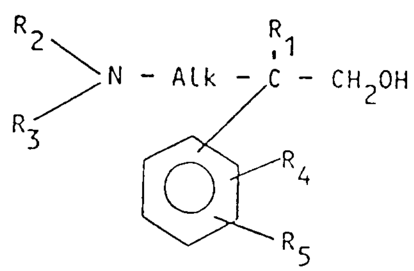

(I)

dans laquelle :

| | |
|---|---|
| Alk | représente un groupe alkylène de 2 à 10 atomes de carbone ; |
| $R_1$ | représente l'hydrogène ou un alkyle comportant de 1 à 6 atomes de carbone ; |
| $R_2$ et $R_3$ | sont semblables ou différents et représentent un cycloalkyle de 3 à 6 atomes de carbone ou un alkyle droit ou ramifié comportant de 1 à 6 atomes de carbone non substitué ou substitué par un groupement phényle ou méthylphényle ; ou $R_2$ et $R_3$ forment ensemble avec l'atome d'azote auquel ils sont liés un hétérocycle monoazoté ne contenant pas d'autre hétéroatome, tel que pyrrolidino, pipéridino, azépino, hexaméthylèneimino, méthyl-4 pipéridino, benzyl-4 pipéridino, phényl-4 pipéridino, tétrahydro-1,2,3,4 isoquinolyl-2 ; |
| $R_4$ | représente un hydrogène, un halogène, un méthyle ou un phényle ; |
| $R_5$ | représente un hydrogène, un halogène ou un méthyle |
| ou $R_4$ et $R_5$ | pris ensemble avec le noyau benzénique auquel ils sont liés constituent un groupe naphtyl-1 ou naphtyl-2 ; |

ainsi que leurs sels pharmaceutiquement acceptables avec des acides minéraux ou organiques.

**2.** Dérivé selon la revendication 1, caractérisé en ce qu'il est le chlorhydrate de (benzyl-4 pipéridino)-4 (naphtyl-1)-2 butanol.

3. Dérivé selon la revendication 1, caractérisé en ce qu'il est le chlorhydrate de N,N-diméthylamino-8 (naphtyl-1)-2 octanol.

4. Dérivé selon la revendication 1, caractérisé en ce qu'il est le chlorhydrate de diéthylamino-8 (naphtyl-1)-2 octanol.

5. Dérivé selon la revendication 1, caractérisé en ce qu'il est le chlorhydrate de (benzyl-4 pipéridino)-4 (dichloro-2,4 phényl)-2 butanol.

6. Dérivé selon la revendication 1, caractérisé en ce qu'il est le chlorhydrate de (benzyl-4 pipéridino)-4 (phényl-4 phényl)-2 butanol.

7. Procédé de préparation d'un dérivé aromatique selon la revendication 1, caractérisé en ce que l'on réduit un composé de formule

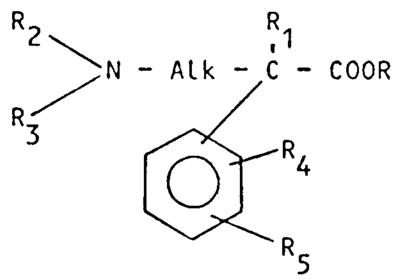

(II)

dans laquelle Alk, $R_1$, $R_2$, $R_3$, $R_4$, $R_5$ sont tels que définis dans la revendication 1 et R représente l'hydrogène ou un groupe alkyle, par un hydrure métallique ou par électrolyse et en ce qu'éventuellement l'on transforme le produit ainsi obtenu en l'un de ses sels pharmaceutiquement acceptables avec des acides minéraux ou organiques.

8. Procédé selon la revendication 7, caractérisé en ce que l'agent réducteur est l'hydrure de sodium bis-(méthoxy-2 éthoxy) aluminium.

9. Composition pharmaceutique à usage externe présentant une activité antimicrobienne et désinfectante, caractérisée en ce qu'elle contient une quantité efficace d'un dérivé aromatique selon la revendication 1.

10. Application des dérivés aromatiques selon la revendication 1, à titre de conservateurs en pharmacie, cosmétologie agroalimentaire.

11. Produits cosmétiques, caractérisés en ce qu'ils contiennent à titre de conservateurs un dérivé aromatique selon la revendication 1.

12. Compositions désinfectantes pour surfaces inertes, caractérisées en ce qu'elles contiennent une quantité efficace d'un dérivé aromatique selon la revendication 1.

13. Compositions biocides utiles dans la récupération améliorée des hydrocarbures, caractérisées en ce qu'elles contiennent une quantité efficace d'un dérivé aromatique selon la revendication 1.

**Revendications pour les Etats contractants suivants : AT, ES**

1. Procédé pour l'obtention de dérivés aromatiques de formule :

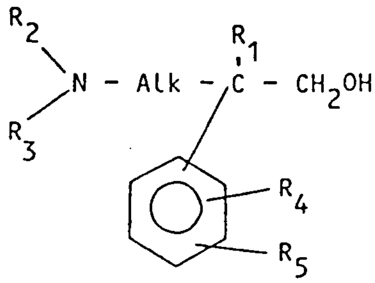

(I)

dans laquelle :

| | |
|---|---|
| Alk | représente un groupe alkylène de 2 à 10 atomes de carbone ; |
| $R_1$ | représente l'hydrogène ou un alkyl comportant de 1 à 6 atomes de carbone ; |
| $R_2$ et $R_3$ | sont semblables ou différents et représentent un cycloalkyle de 3 à 6 atomes de carbone ou un alkyle droit ou ramifié comportant de 1 à 6 atomes de carbone non substitué ou substitué par un groupement phényle ou méthylphényle ; ou $R_2$ et $R_3$ forment ensemble avec l'atome d'azote auquel ils sont liés un hétérocycle monoazoté ne contenant pas d'autre hétéroatome, tel que pyrrolidino, pipéridino, azépino, hexaméthylèneimino, méthyl-4 pipéridino, benzyl-4 pipéridino, phényl-4 pipéridino, tétrahydro-1,2,3,4 isoquinolyl-2 ; |
| $R_4$ | représente un hydrogène, un halogène, un méthyle ou un phényle ; |
| $R_5$ | représente un hydrogène, un halogène ou un méthyle ; |
| ou $R_4$ et $R_5$ | pris ensemble avec le noyau benzénique auquel ils sont liés constituent un groupe naphtyl-1 ou naphtyl-2; |

ainsi que de leurs sels pharmaceutiquement acceptables avec des acides minéraux ou organiques, caractérisé en ce qu'il consiste à réduire un composé de formule :

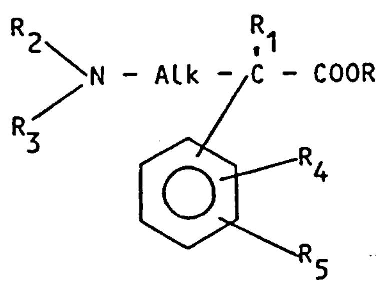

(II)

dans laquelle Alk, $R_1$, $R_2$, $R_3$, $R_4$ et $R_5$ sont tels que définis ci-dessus et R représente l'hydrogène ou un groupe alkyle, par un hydrure métallique ou par électrolyse et en ce qu'éventuellement l'on transforme le produit ainsi obtenu en l'un de ses sels pharmaceutiquement acceptables avec des acides minéraux ou organiques.

**2.** Procédé selon la revendication 1, caractérisé en ce que l'agent réducteur est l'hydrure de sodium bis-(méthoxy-2 éthoxy) aluminium.

**3.** Procédé selon l'une des revendications 1 ou 2, caractérisé en ce que l'on réduit un composé de formule II dans laquelle $R_1$ est l'hydrogène, $R_2$ et $R_3$ forment ensemble le groupe benzyl-4 pipéridino, Alk est le groupe $-(CH_2)_2-$ et $R_4$ et $R_5$ forment ensemble avec le noyau benzénique auquel ils sont liés le groupe naphtyl-1.

**4.** Procédé selon l'une des revendications 1 ou 2, caractérisé en ce que l'on réduit un composé de formule II dans laquelle $R_1$ représente l'hydrogène ; $R_2$ et $R_3$ sont chacun le groupe méthyle; $R_4$ et $R_5$ forment ensemble avec le noyau benzénique auquel ils sont liés le groupe naphtyl-1 ; Alk est le groupe $-(CH_2)_6-$.

**5.** Procédé selon l'une des revendications 1 ou 2, caractérisé en ce que l'on réduit un composé de

formule II dans laquelle $R_1$ représente l'hydrogène, $R_2$ et $R_3$ sont chacun le groupe éthyle ; $R_4$ et $R_5$ forment ensemble avec le noyau benzénique auquel ils sont liés le groupe naphtyl-1 ; Alk est le groupe $-(CH_2)_6-$.

**6.** Procédé selon l'une des revendications 1 ou 2, caractérisé en ce que l'on réduit un composé de formule II dans laquelle $R_2$ et $R_3$ forment ensemble le groupe benzyl-4 pipéridino ; $R_4$ et $R_5$ sont le groupe dichloro-2,4 ; Alk est le groupe $-(CH_2)_2$ ; $R_1$ est l'hydrogène.

**7.** Procédé selon l'une des revendications 1 ou 2, caractérisé en ce que l'on réduit un composé de formule II dans laquelle $R_2$ et $R_3$ forment ensemble le groupe benzyl-4 pipéridino ; $R_5$ est l'hydrogène et $R_4$ est un groupe phényl-4 ; $R_1$ est l'hydrogène et Alk est le groupe $-(CH_2)_2-$.

**8.** Utilisation d'une quantité efficace d'un dérivé aromatique de formule (I), obtenu selon l'une quelconque des revendications 1 à 7, pour la préparation de compositions pharmaceutiques à usage externe, présentant une activité antimicrobienne et désinfectante.

**9.** Application des dérivés aromatiques de formule (I), obtenus selon l'une quelconque des revendications 1 à 7, à titre de conservateurs en pharmacie, cosmétologie, agroalimentaire.

**10.** Produits cosmétiques, caractérisés en ce qu'ils contiennent à titre de conservateurs un dérivé aromatique de formule (I), obtenu selon l'une quelconque des revendications 1 à 7.

**11.** Compositions désinfectantes pour surfaces inertes, caractérisées en ce qu'elles contiennent une quantité efficace d'un dérivé aromatique de formule (I), obtenu selon l'une quelconque des revendications 1 à 7.

**12.** Compositions biocides utiles dans la récupération améliorée des hydrocarbures, caractérisées en ce qu'elles contiennent une quantité efficace d'un dérivé aromatique de formule (I), obtenu selon l'une quelconque des revendications 1 à 7.

## Claims

**Claims for the following Contracting States : BE, CH, DE, FR, GB, IT, LI, LU, NL, SE**

**1.** Aromatic derivatives of formula:

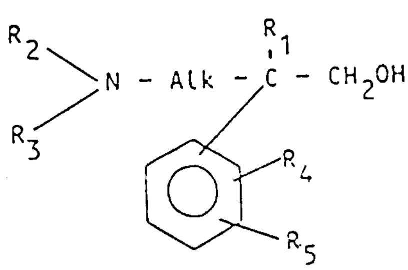

(I)

wherein:

- Alk is an alkylene group with 2 to 10 carbon atoms,
- $R_1$ is hydrogen or an alkyl with 1 to 6 carbon atoms,
- $R_2$ and $R_3$ are similar or different and represent a cycloalkyl with 3 to 6 carbon atoms or a straight or branched alkyl with 1 to 6 carbon atoms, either non-substituted or substituted by a phenyl or methyl -phenyl group, or $R_2$ and $R_3$ form, together with the atom of nitrogen to which they are bonded, a mono-nitrogenous heterocycle containing no other heteroatom, such as pyrrolidino, piperidino, azepino, hexamethyleneimino, 4-methylpiperidino, 4-benzylpiperidino, 4-phenylpiperidino, 1,2,3,4-tetrahydro-2-isoquinolyl,
- $R_4$ is hydrogen, halogen, methyl or phenyl,
- $R_5$ is hydrogen, halogen or methyl,

- or $R_4$ and $R_5$ together with the benzene ring to which they are bonded, constitute a 1-naphthyl or 2-naphthyl group,

and the pharmaceutically acceptable salts with mineral or organic acids.

**2.** Derivative according to claim 1, characterized in that it is 4-(4-benzylpiperidino)-2-(1-naphthyl) butanol hydrochloride.

**3.** Derivative according to claim 1, characterized in that it is 8-(N,N-dimethylamino)-2-(1-naphthyl) octanol hydrochloride.

**4.** Derivative according to claim 1, characterized in that it is 8-diethylamino-2-(1-naphthyl)octanol hydrochloride.

**5.** Derivative according to claim 1, characterized in that it is 4-(4-benzylpiperidino)-2-(2,4-dichlorophenyl)-butanol hydrochloride.

**6.** Derivative according to claim 1, characterized in that it is 4-(4-benzylpiperidino)-2-(4-phenyl phenyl)-butanol hydrochloride.

**7.** Process for the preparation of an aromatic derivative according to claim 1, characterized in that it consists in reducing a compound of formula:

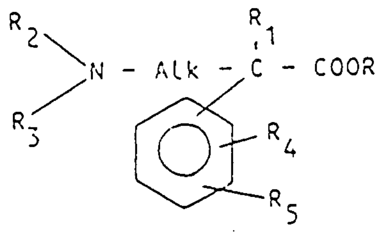

(II)

in which Alk, $R_1$, $R_2$, $R_3$, $R_4$, $R_5$ are as defined in claim 1 and R is hydrogen or an alkyl group, with a metal hydride or by electrolysis, and in that the resulting product is then optionally converted into one of its pharmaceutically acceptable salts with mineral or organic acids.

**8.** Process according to claim 7, characterized in that the reducing agent is sodium bis(2-methoxyethoxy)-aluminium hydride.

**9.** Pharmaceutical composition for external use, having an antimicrobial and disinfectant activity, characterized in that it contains an effective quantity of aromatic derivative according to claim 1.

**10.** Application of the aromatic derivatives according to claim 1, as preservatives in pharmacy, cosmetology, and agri-foodstuffs.

**11.** Cosmetic products, characterized in that they contain as preservatives an aromatic derivative according to claim 1.

**12.** Disinfectant compositions for inert surfaces, characterized in that they contain an effective quantity of an aromatic derivative according to claim 1.

**13.** Biocidal compositions for use in the improved recovery of hydrocarbons, characterized in that said compositions contain an effective quantity of an aromatic derivative according to claim 1.

**Claims for the following Contracting States : AT, ES**

**1.** Process for the preparation of aromatic derivatives of formula:

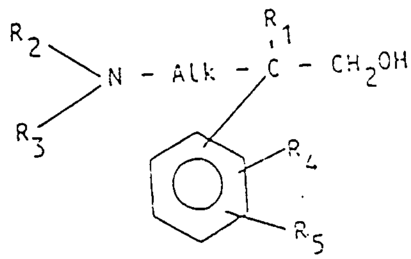

(I)

wherein:

- Alk is an alkylene group with 2 to 10 carbon atoms,
- $R_1$ is hydrogen or an alkyl comprising 1 to 6 carbon atoms,
- $R_2$ and $R_3$ are similar or different and represent a cycloalkyl with 3 to 6 carbon atoms or a straight or branched alkyl with 1 to 6 carbon atoms, either non-substituted or substituted by a phenyl or methyl phenyl group, or $R_2$ and $R_3$ form, together with the atom of nitrogen to which they are bonded, a mono-nitrogenous heterocycle containing no other heteroatom, such as pyrrolidino, piperidino, azepino, hexamethyleneimino, 4-methylpiperidino, 4-benzylpiperidino, 4-phenylpiperidino, 1,2,3,4-tetrahydro-2-isoquinolyl;
- $R_4$ is hydrogen, halogen, methyl or phenyl,
- $R_5$ is hydrogen, halogen or methyl,
- or $R_4$ and $R_5$ together with the benzene ring to which they are bonded, constitute a 1-naphthyl or 2-naphthyl group,

and the pharmaceutically acceptable salts with mineral or organic acids, characterized in that it consists in reducing a compound of formula:

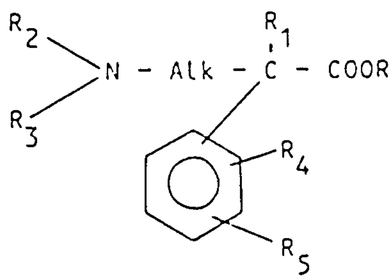

(II)

in which Alk, $R_1$, $R_2$, $R_3$, $R_4$ and $R_5$ are as defined above and R is hydrogen or an alkyl group, with a metal hydride or by electrolysis, and in that the resulting product is then optionally converted into one of its pharmaceutically acceptable salts with mineral or organic acids.

**2.** Process according to claim 1, characterized in that the reducing agent is sodium bis(2-methoxyethoxy)-aluminium hydride.

**3.** Process according to one of claims 1 or 2, characterized in that it consists in reducing a compound of formula II in which $R_1$ is hydrogen, $R_2$ and $R_3$ form together the 4-benzylpiperidino group, Alk is the $-(CH_2)_2$-group and $R_4$ and $R_5$ form, together with the benzene ring to which they are bonded, the 1-naphthyl group.

**4.** Process according to one of claims 1 or 2, characterized in that a compound of formula II is reduced, wherein $R_1$ is hydrogen ; $R_2$ and $R_3$ are each the methyl group ; $R_4$ and $R_5$ form, together with the benzene ring to which they are bonded, the 1-naphthyl group; Alk is the $-(CH_2)_6$-group.

**5.** Process according to one of claims 1 or 2, characterized in that it consists in reducing a compound of formula II in which $R_1$ is hydrogen ; $R_2$ and $R_3$ are each the ethyl group; $R_4$ and $R_5$ form, together with the benzene ring to which they are bonded, the 1-naphthyl benzene ring to which they are bonded, the 1-naphthyl group ; Alk is the $-(CH_2)_6$-group.

6. Process according to one of claims 1 or 2, characterized in that it consists in reducing a compound of formula II in which $R_2$ and $R_3$ form together the 4-benzylpiperidino group ; $R_4$ and $R_5$ are the 2,4-dichloro group ; Alk is the $-(CH_2)_2$-group ; $R_1$ is hydrogen.

7. Process according to one of claims 1 or 2, characterized in that it consists in reducing a compound of formula II in which $R_2$ and $R_3$ form together the 4-benzylpiperidino group ; $R_5$ is hydrogen and $R_4$ is a 4-phenyl group ; $R_1$ is hydrogen and Alk is the $-(CH_2)_2$-group.

8. Use of an effective quantity of an aromatic derivative of formula (I), obtained according to any one of claims 1 to 7, for the preparation of pharmaceutical compositions for external use, having an antimicrobial and disinfectant activity.

9. Application of the aromatic derivatives of formula (I), obtained according to any one of claims 1 to 7, as preservatives in pharmacy, cosmetology, and agri-foodstuffs.

10. Cosmetic products, characterized in that they contain as preservatives an aromatic derivative of claims 1 to 7.

11. Disinfectant compositions for inert surfaces, characterized in that they contain an effective quantity of an aromatic derivative of formula (I) obtained according to any one of claims 1 to 7.

12. Biocidal compositions for use in the improved recovery of hydrocarbons, characterized in that they contain an effective quantity of an aromatic derivative of formula (I), obtained according to any one of claims 1 to 7.

**Patentansprüche**
**Patentansprüche für folgende Vertragsstaaten : BE, CH, DE, FR, GB, IT, LI, LU, NL, SE**

1. Aromatische Derivate der Formel

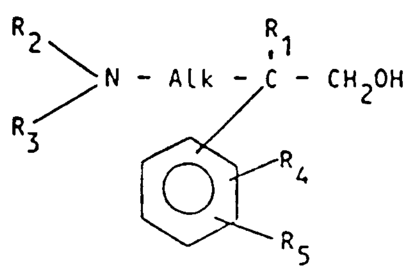

(I)

in welcher:

| | |
|---|---|
| Alk | eine Alkylengruppe mit 2 bis 10 Kohlenstoffatomen darstellt; |
| $R_1$ | Wasserstoff oder Alkyl mit 1 bis 6 Kohlenstoffatomen darstellt; |
| $R_2$ und $R_3$ | gleich oder verschieden sind und ein Cycloalkyl mit 3 bis 6 Kohlenstoffatomen oder ein gerades oder verzweigtes nichtsubstituiertes oder durch eine Phenyl- oder Methylphenylgruppe substituiertes Alkyl mit 1 bis 6 Kohlenstoffatomen darstellen; oder $R_2$ und $R_3$, zusammen mit dem Stickstoffatom, an das sie gebunden sind, einen monoazotierten Heterocyclus bilden, der kein anderes Heteroatom enthält, wie Pyrrolidino, Piperidino, Azepino, Hexamethylenimino, 4-Methyl-piperidino, 4-Benzyl-piperidino, 4-Phenyl-piperidino, 1,2,3,4-Tetrahydro-isochinol-2-yl; |
| $R_4$ | Wasserstoff, ein Halogen, Methyl oder Phenyl darstellt; |
| $R_5$ | Wasserstoff, ein Halogen oder Methyl darstellt, oder |
| $R_4$ und $R_5$, | zusammen mit dem Benzolkern, an den sie gebunden sind, eine 1-Naphthyl- oder 2-Naphthylgruppe darstellen; |

sowie deren pharmazeutisch akzeptable Salze mit Mineral- oder organischen Säuren.

2. Derivat nach Anspruch 1, dadurch gekennzeichnet, daß es 4-(4-Benzyl-piperidino)-2-(1-naphthyl)-butanol-hydrochlorid ist.

3. Derivat nach Anspruch 1, dadurch gekennzeichnet, daß es 8-N,N-Dimethylamino-2-(1-naphthyl)-octanol-hydrochlorid ist.

4. Derivat nach Anspruch 1, dadurch gekennzeichnet, daß es 8-N,N-Diethylamino-2-(1-naphthyl)-octanol-hydrochlorid ist.

5. Derivat nach Anspruch 1, dadurch gekennzeichnet, daß es 4-(4-Benzyl-piperidino)-2-(2,4-dichlorphenyl)-butanol-hydrochloridist.

6. Derivat nach Anspruch 1, dadurch gekennzeichnet, daß es 4-(4-Benzyl-piperidino)-2-(4-phenylphenyl)-butanol-hydrochlorid ist.

7. Verfahren zur Herstellung eines aromatischen Derivats nach Anspruch 1, dadurch gekennzeichnet, daß man eine Verbindung der Formel

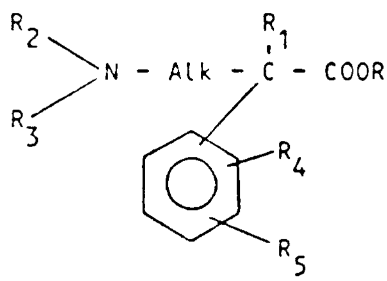

(II)

in welcher Alk, $R_1$, $R_2$, $R_3$, $R_4$, $R_5$ wie in Anspruch 1 definiert sind und R für Wasserstoff oder eine Alkylgruppe steht, mit einem Metallhydrid oder durch Elektrolyse reduziert, und daß man gegebenenfalls das so erhaltene Produkt in eines seiner pharmazeutisch akzeptablen Salze mit Mineral- oder organischen Säuren überführt.

8. Verfahren nach Anspruch 7, dadurch gekennzeichnet, daß das Reduktionsmittel Natrium-bis-(2-methoxyethoxy)aluminiumhydrid ist.

9. Pharmazeutische Zusammensetzung für äußere Anwendung mit antimikrobieller und desinfizierender Aktivität, dadurch gekennzeichnet, daß sie eine wirksame Menge eines aromatischen Derivats nach Anspruch 1 enthält.

10. Anwendung der aromatischen Derivate nach Anspruch 1 als Konservierungsmittel in der Pharmazie, Kosmetologie und agroalimentären Industrie.

11. Kosmetikprodukte, dadurch gekennzeichnet, daß sie als Konservierungsmittel ein aromatisches Derivat nach Anspruch 1 enthalten.

12. Desinfektionszusammensetzungen für inerte Oberflächen, dadurch gekennzeichnet, daß sie eine wirksame Menge eines aromatischen Derivats nach Anspruch 1 enthalten.

13. Biozide Zusammensetzungen, die bei der verbesserten Rückgewinnung von Kohlenwasserstoffen nützlich sind, dadurch gekennzeichnet, daß sie eine wirksame Menge eines aromatischen Derivats nach Anspruch 1 enthalten.

**Patentansprüche für folgende Vertragsstaaten : AT, ES**

1. Verfahren zur Herstellung von aromatischen Derivaten der Formel

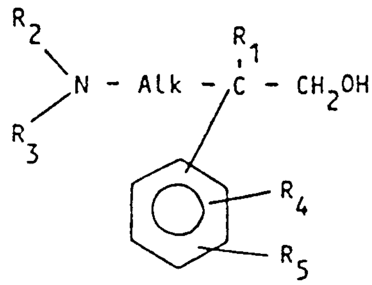

(I)

in welcher:

| | |
|---|---|
| Alk | eine Alkylengruppe mit 2 bis 10 Kohlenstoffatomen darstellt; |
| $R_1$ | Wasserstoff oder Alkyl mit 1 bis 6 Kohlenstoffatomen darstellt; |
| $R_2$ und $R_3$ | gleich oder verschieden sind und ein Cycloalkyl mit 3 bis 6 Kohlenstoffatomen oder ein gerades oder verzweigtes nichtsubstituiertes oder durch eine Phenyl- oder Methylphenylgruppe substituiertes Alkyl mit 1 bis 6 Kohlenstoffatomen darstellen; oder $R_2$ und $R_3$, zusammen mit dem Stickstoffatom, an das sie gebunden sind, einen monoazotierten Heterocyclus bilden, der kein anderes Heteroatom enthält, wie Pyrrolidino, Piperidino, Azepino, Hexamethylenimino, 4-Methyl-piperidino, 4-Benzyl-piperidino, 4-Phenyl-piperidino, 1,2,3,4-Tetrahydro-isochinol-2-yl; |
| $R_4$ | Wasserstoff, ein Halogen, Methyl oder Phenyl darstellt; |
| $R_5$ | Wasserstoff, ein Halogen oder Methyl darstellt, oder |
| $R_4$ und $R_5$, | zusammen mit dem Benzolkern, an den sie gebunden sind, eine 1-Naphthyl- oder 2-Naphthylgruppe darstellen; |

sowie von deren pharmazeutisch akzeptablen Salzen mit Mineral- oder organischen Säuren, dadurch gekennzeichnet, daß es darin besteht, daß eine Verbindung der Formel

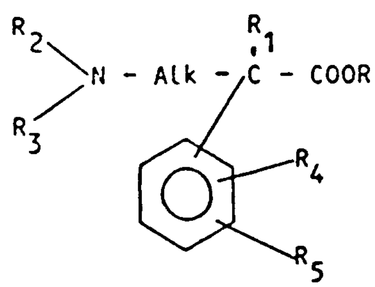

(II)

in welcher Alk, $R_1$, $R_2$, $R_3$, $R_4$, $R_5$ wie oben definiert sind und R für Wasserstoff oder eine Alkylgruppe steht, mit einem Metallhydrid oder durch Elektrolyse reduziert wird, und daß gegebenenfalls das so erhaltene Produkt in eines seiner pharmazeutisch akzeptablen Salze mit Mineral- oder organischen Säuren übergeführt wird.

**2.** Verfahren nach Anspruch 1, dadurch gekennzeichnet, daß das Reduktionsmittel Natrium-bis-(2-methoxyethoxy)aluminiumhydrid ist.

**3.** Verfahren nach einem der Ansprüche 1 oder 2, dadurch gekennzeichnet, daß man eine Verbindung von II reduziert, in welcher $R_1$ Wasserstoff ist, $R_2$ und $R_3$ zusammen die Gruppe 4-Benzyl-piperidino bilden, Alk für die Gruppe $-(CH_2)_2-$steht und $R_4$ und $R_5$, zusammen mit dem Benzolkern, an den sie gebunden sind, 1-Naphthyl bilden.

**4.** Verfahren nach einem der Ansprüche 1 oder 2, dadurch gekennzeichnet, daß man eine Verbindung der Formel II reduziert, in welcher $R_1$ Wasserstoff darstellt; $R_2$ und $R_3$ jeweils Methyl sind; $R_4$ und $R_5$,

zusammen mit dem Benzolkern, an den sie gebunden sind, 1-Naphthyl bilden und Alk die Gruppe $-(CH_2)_6-$ ist.

**5.** Verfahren nach einem der Ansprüche 1 oder 2, dadurch gekennzeichnet, daß man eine Verbindung der Formel II reduziert, in welcher $R_1$ Wasserstoff darstellt; $R_2$ und $R_3$ jeweils Ethyl sind; $R_4$ und $R_5$, zusammen mit dem Benzolkern, an den sie gebunden sind, 1-Naphthyl bilden und Alk die Gruppe $-(CH_2)_6-$ ist.

**6.** Verfahren nach einem der Ansprüche 1 oder 2, dadurch gekennzeichnet, daß man eine Verbindung der Formel II reduziert, in welcher $R_2$ und $R_3$ zusammen die Gruppe 4-Benzylpiperidino bilden; $R_4$ und $R_5$ die Gruppe 2,4-Dichlor sind; Alk die Gruppe $-(CH_2)_2$ ist und $R_1$ für Wasserstoff steht.

**7.** Verfahren nach einem der Ansprüche 1 oder 2, dadurch gekennzeichnet, daß man eine Verbindung der Formel II reduziert, in welcher $R_2$ und $R_3$ zusammen die Gruppe 4-Benzylpiperidino bilden; $R_5$ Wasserstoff ist und $R_4$ 4-Phenyl ist; $R_1$ für Wasserstoff steht und Alk die Gruppe $-(CH_2)_2$ ist.

**8.** Verwendung einer wirksamen Menge eines aromatischen Derivats der Formel (I), erhalten nach einem der Ansprüche 1 bis 7, zur Herstellung von pharmazeutischen Zusammensetzungen für äußere Anwendung mit antimikrobieller und desinfizierender Aktivität.

**9.** Anwendung der aromatischen Derivate der Formel (I), erhalten nach einem der Ansprüche 1 bis 7, als Konservierungsmittel in der Pharmazie, Kosmetologie und agroalimentären Industrie.

**10.** Kosmetikprodukte, dadurch gekennzeichnet, daß sie als Konservierungsmittel ein aromatisches Derivat der Formel (I), erhalten nach einem der Ansprüche 1 bis 7, enthalten.

**11.** Desinfektionszusammensetzungen für inerte Oberflächen, dadurch gekennzeichnet, daß sie eine wirksame Menge eines aromatischen Derivats der Formel (I), erhalten nach einem der Ansprüche 1 bis 7, enthalten.

**12.** Biozide Zusammensetzungen, die bei der verbesserten Rückgewinnung von Kohlenwasserstoffen nützlich sind, dadurch gekennzeichnet, daß sie eine wirksame Menge eines aromatischen Derivats der Formel (I), erhalten nach einem der Ansprüche 1 bis 7, enthalten.